# EUROPEAN PATENT APPLICATION

(11) **EP 1 736 161 A2**
(43) Date of publication of application: **27.12.2006**
(21) Application number: 06012727.1
(22) Date of filing: 21.06.2006
(51) Int. Cl.: A61K 33/36, A61P 11/06, A61P 37/00, A61P 33/02, A61P 33/06

(54) **Use of an arsenic compound for the manufacture of a medicament for the treatment of arsenic susceptible blast-like cell related diseases**

(30) Priority: 24.06.2005 US 165454
(71) Applicant: Hsin, Shao-Chi, Taipei (TW)
(72) Inventor: Hsin, Shao-Chi, Taipei (TW)
(74) Representative: Casalonga, Axel

(57) **Abstract**

Use of an arsenic compound for the manufacture of a medicament for treatment of arsenic-susceptible blast-like cell related diseases, such as hypersusceptible diseases, immune or autoimmune diseases, fibroblast-related diseases, inflammation diseases or parasitic diseases.

## Description

### 1. Field of Invention

The present invention is related to use an arsenic compound for the manufacture of a medicament for the treatment of arsenic susceptible blast-like cell related diseases including malaria, Trypanosoma, bronchial asthma and systemic lupus erythematosus.

### 2. Description of the Related Art

Malaria, Trypanosoma, bronchial asthma and systemic lupus erythematosus are widespread around the world. People spent a lot of money for searching a common drug to treat those diseases. However, no drug was developed specifically for the common treatment of the diseases.

### Systemic lupus erythematosus

Systemic lupus erythematosus (SLE) is a systemic autoimmune disease with the potential to be directly involved in multiple organ systems. The clinical manifestations of SLE include skin rash, joint pain and severe and progressive .kidney involvement.

Several years ago, an animal model of SLE was established in the laboratory. Based on the concept of the idiotypic network, animals developed a wide spectrum of lupus-related autoantibodies and clinical manifestations in this model. The induction was carried out by the immunization of mouse strains that had not developed any spontaneous autoimmune disorders before the induction, with a human anti-DNA monoclonal antibody (mAb) which bears a common idiotype termed 16/6 Id. The serological findings were associated with leukopenia, elevated erythrocyte sedimentation rate, proteinuria, abundance of immune complexes in the kidneys and sclerosis of the glomeruli, which are typical manifestations of SLE. The induction of the disease is genetically controlled, and thus is strain dependent. This unique model for the induction of experimental SLE provides an appropriate tools to clearly dissect the different steps and the linked immune parameters involved in the induction and development of SLE.

A majority of patients with SLE have symptoms of kidney failure. Clinical presentations typically include asymptomatic hematuria or proteinuria, acute nephritic or nephrotic syndromes, rapidly progressive glomerulonephritis and chronic renal insufficiency.

A number of therapeutic treatments have addressed lupus nephritis, although commonly available therapeutic regimes are potentially toxic and may be ineffective in some high risk patients. For severe SLE, immunosuppressives such as chemotherapies and cyclosporin are usually used. Other treatments include administering corticosteroids and cytotoxic drugs, as well as cyclophosphamide and prednisone. Side effects of long term use of prednisone include development of high blood pressure, diabetes and osteoporosis. Many pharmaceutical companies are searching for treating SLE.

### Malaria

Malaria is an infectious parasitic disease transmitted by mosquitoes, and is characterized by a periodic fever and enlarged spleen. Malaria affects some 200 million people around the world a year. Malaria in humans is caused by 4 species of parasitic protozoa belonging to the genus *Plasmodium.* Of these, *P. falciparum* could induce severe disease while *P. malariae, P. vivax* and *P. ovale* cause milder forms.

Malaria is transmitted by infected female *Anopheline mosquitoes.* The *Plasmodia* parasite matures in the insect, and is then transferred when the mosquito bites a human. Inside the human, the parasite settles first in the liver, multiplies and then invades the red blood cells.

Despite numerous attempts at eradication, malaria remains a serious endemic disease in many areas of Africa, Latin America and Oceania, with a worldwide mortality rate of approximately 1 million people per year. One of the major factors attributing to the continued presence of malaria is malaria parasite that are resistant to one or more anti-malarial drugs.

Mefloquine is known to be effective against species of *Plasmodium* which have developed resistance to conventional anti-malarial agents. However, mefloquine resistance has now been reported in a number of areas in the world. Nevertheless, mefloquine is still one of the most effective anti-malarial mono-therapies and its use has increased greatly. Recently, the drug has attracted considerable adverse publicity owing to the incidence of severe neuropsychiatric side-effects, e.g. depression, psychosis, panic attacks and generalized anxiety.

### Trypanosoma

The disease commonly known as sleeping sickness is caused by the parasite *Trypanosoma brucei.* This disease, which is transmitted to humans through the bite of the tsetse fly, has been estimated to affect 300-500 thousand people and may be fatal if left untreated.

*Trypanosoma brucei* exists as two subspecies, *Trypanosoma brucei rhodesiense* and *Trypanosoma brucei gambiense.* Each subspecies develops within the tsetse fly and the metacyclic trypomastigote is passed into the bloodstream of its victim through the salivary gland of the fly following a bite. Once in the blood stream the parasite spreads rapidly throughout the host. The initial infection stage involves infection of the lymph system, and results in enlarged lymph nodes, headache and irregular fevers. The second stage involves invasion of the central nervous system with neurological symptoms of progressive mental apathy, extended daytime sleeping, and loss of appetite. These neurological effects along with concurrent involvement of the muscular system can result in progression to paralysis and irreversible coma.

The parasite cable of invading the central nervous system has required two separate treatments due to the difficulty of drug penetration through the blood-brain barrier. Each of the treatments requires drug delivery through injections. Intramuscular injections are required for either pentamidine isethionate or suramine sodium for the early stage of infection. The second stage of infection, involving the central nervous system, requires intravenous injections of either melarsoprol or eflornithine. Serious side effects include hypotension, abdominal pain, vertigo, hypersalivation and mild nephrotoxicity by pentamindine isethionate treatment. Nausea, vomiting, uticaria and possible renal damages or exfoliative dermatitis occur when using suramine sodium. The development of new drugs for treating sleeping sickness has been very slow. For example, the commonly used second stage drug melarsoprol was developed in 1932 and is a highly toxic arsenic-based molecule. This drug can cause myocardial damage, hypertension, exfoliative dermatitis and reactive encephalopathy, which occurs in 5-10% of the patients and can lead to death. Eflornithine, an inhibitor of the enzyme ornithine decarboxylase, is the only drug suitable for patients where melarsoprol is ineffective but it is poorly effective against *Trypanosome brucei rhodesiense.* This drug causes mild side effects such as diarrhea, anemia, thrombocytopenia, vomiting and fever.

Recently, *Trypanosoma brucei* resistant strains to these drugs have been identified. For this reason and due to the few treatment options it is important to develop new therapeutic strategies for treating this disease. In an effort to identify a new and more effective method for treating *Trypanosoma brucei* Gelb *et al.* reported the successful use of prenyl transferase inhibitors in inhibiting the growth of *Trypanosoma brucei* parasites.

In view of the need to find new treatments for *Trypanosoma brucei* infections, those skilled in the art would welcome an effective method for the treatment utilizing inhibitors of *Trypanosoma brucei* prenyl transferases.

### Bronchial asthma

Bronchial asthma was regarded as an abnormality of respiratory smooth muscle in which afflicted individuals experienced the onset of bronchospasm as a consequence of over-reactivity of the bronchial smooth muscle for many years.

### Arsenic and its medical uses

Arsenic has been considered to be both a poison and a drug for a long time in both Western and Chinese medical practices. In the latter part of the nineteenth century, arsenic was used frequently in attempts to treat diseases of the blood in the West, In traditional Chinese medicine, arsenous acid or arsenic trioxide paste has been used to treat tooth marrow diseases, psoriasis, syphilis and rheumatosis.

At a minimum, a need still exists in the related art to treat the foregoing diseases and display a desired effect.

An aspect of the present invention is related to use of an arsenic compound for the manufacture of a medicament for treatment of arsenic-susceptible blast-like cell related diseases.

Preferably, said arsenic compound is administered parenterally.

Preferably, said arsenic-susceptible blast-like cell related disease is a hypersusceptible disease, an immune or autoimmune disease, a fibroblast-related disease, an inflammation disease and a parasitic disease.

More preferably, the immune or autoimmune disease suitable for the use according to the present invention is a connective tissue disease, an autoimmune thyroid disease, a neuromuscular junction autoimmune disease, an autoimmune gastrointestinal disease, autoimmune hepatitis, primary biliary cirrhosis, primary sclerosing cholangitis, autoimmune carditis or arteritis.

More preferably, the immune or autoimmune disease suitable for the use according to the present invention is systemic lupus erythematosus, rheumatoid arthritis, sclerosis, Graves' disease, Myasthenia Gravis, multiple sclerosis, ulcerative colitis or Crohn's disease.

Preferably, the hypersusceptible disease suitable for the use according to the present invention is bronchial asthma, hypersensitivity pneumonitis, diffuse pulmonary interstitial fibrosis, allergic rhinitis, eosinophil-associated nasal inflammation, vernal conjunctivitis and urticaria.

Preferably, the inflammation disease suitable for the use according to the present invention is pneumoconiosis, osteomyelitis, leprous nodule, syphilis, tuberculosis, hepatitis, tumor formation, a chronic obstructive pulmonary disease.

Preferably, the fibroblast-related disease suitable for the use according to the present invention is hepatic fibrosis, pulmonary fibrosis, cutaneous and subcutaneous fibrosis and systemic fibrosis.

More preferably, the fibroblast-related disease suitable for the use according to the present invention is liver cirrhosis, pneumoconiosis, tuberculosis, severe acute respiratory syndrome (SARS), adult (acute) respiratory distress syndrome (ARDS), a chronic obstructive pulmonary disease (COPD), scarring, keloid, psoriasis, cystic fibrosis or neurofibromatosis.

Preferably, the parasitic disease suitable for the use according to the present invention is malaria or trypanosomiasis.

Preferably, the arsenic-susceptible blast-like cell is blast-like cell of a white blood cell, a peripheral blood mononuclear cell, a fibroblast and a parasite.

Preferably, the arsenic compound is administered is in a dosage of between 0.001 micromolar to 20 micromolar.

More preferably, the arsenic compound is administered is in a dosage of between 0.1 micromolar to 15 micromolar.

Most preferably, the arsenic compound is administered is in a dosage of between 0.1 micromolar to 10 micromolar.

Other objectives, advantages and novel features of the invention will become more apparent from the following detailed description when taken in conjunction with the accompanying drawings.

### IN THE DRAWINGS

Fig. 1 is a bar chart showing the result of MTT assay for human fibroblasts exposed in arsenic trioxide; and
Fig. 2 is a bar chart showing the result of the biological effect of arsenic trioxide on cell cycle.

The present invention provides a method for treating inflammation diseases, comprising administering to a human or animal afflicted with such a disease, an amount of pharmaceutically acceptable arsenic compounds, wherein said amount is effective to counteract the symptoms of inflammation diseases.

The present invention provides a method for treating fibroblast-related diseases, comprising administering to a human or animal afflicted with such a disease, an amount of pharmaceutically acceptable arsenic compounds, wherein said amount is effective to counteract the symptoms of fibroblast-related diseases.

The present invention provides a method for treating malaria and trypanosomiasis, as well as other parasitic diseases, comprising administering to a human or animal afflicted with such a disease, an amount of pharmaceutically acceptable arsenic compounds, wherein said amount is effective to counteract the symptoms of malaria, trypanosomiasis or other diseases.

The parasites induced malaria and trypanosomiasis proliferate by asexual reproduction in human body. It is believed that the arsenic compounds can reduce the infection rates of malaria and trypanosomiasis. A preferred method of administering the arsenic compounds is parenterally, as by injection, infusion, topical application or inhalation.

The present invention provides a method for treating systemic lupus erythematosus, as well as other immune or autoimmune diseases, comprising administering to a human or animal afflicted with such a disease, an amount of pharmaceutically acceptable arsenic compounds, wherein said amount is effective to counteract the symptoms of systemic lupus erythematosus or other diseases.

SLE patients typically present elevated serum levels of antibodies against nuclear constituents (i.e., antinuclear antibodies). Specific T helper cells (Th cells) may initiate and sustain the production of pathogenic, anti-nuclear autoantibodies during the onset and progression of systemic lupus erythematosus (SLE) through cognate interaction with autoimmune B cells. These SLE-associated Th cells are primarily responsible for driving the pathogenic autoimmune response. Without the help provided by these Th cells, autoimmune B cells are unable to produce the disease-causing (pathogenic) autoantibodies associated with SLE.

It is believed that the arsenic compounds can induce the apoptosis of Th cells and autoimmune B cells that associated with SLE. A preferred method of administering the arsenic compounds is parenterally, as by injection, infusion, topical application or inhalation.

The present invention provides a method for treating bronchial asthma, as well as other hypersensitivity diseases, comprising administering to a human or animal afflicted with such a disease, an amount of pharmaceutically acceptable arsenic compounds, wherein said amount is effective to counteract the symptoms of bronchial asthma or other diseases.

In the mid 1970's, it was demonstrated that the severity of bronchial asthma can be related to the number of eosinophils in the peripheral blood of the patients. Also around that time, studies of eosinophils had shown the presence of basic (cationic) granule proteins. One of the principal proteins associated with eosinophil granules, the major basic protein (MBP), was so-named because in the guinea pig it comprises more than 50% of the granule protein, is strongly basic (arginine-rich), and is proteinaceous. The prominent role of eosinophils as effector cells in asthma and allergy has contributed to considerable interest in the mechanisms that are involved in the recruitment of these cells. Of particular importance are the chemical signals released in the lungs that initiate and orchestrate the process of eosinophil recruitment from the blood microvessels in the airway wall. Some scientists lavaged guinea pig airways at various intervals after allergen challenge, injected the bronchoalveolar lavage (BAL) fluid intradermally for guinea pigs that were previously injected intravenously with In-eosinophils, and measured eosinophil accumulation in the skin sites. Using this technique, eosinophil chemoattractant activity was detected in the BAL fluid with a peak at 3 to 6 hr after challenge. This activity was purified in a series of high-performance liquid chromatography steps, using the *in vivo* skin bioassay at each stage in order to locate the chemoattractant. Microsequencing revealed a novel 73 amino acid C-C chemokine that is termed here as 'eotaxin' (condensed from eosinophil chemotaxin). The chemokine was present in three fractions at the final reversed phase high-performance liquid chromatography purification stage, which are believed to correspond to three glycosylation variants of eotaxin.

The small protein, eotaxin, is known to be synthesized by a number of different cell types, and is stimulated by interleukin-4 and interleukin-13, which are produced by T-helper (Th) 2 lymphocytes. If eotaxin amount can be reduced, stimulation by eotaxin will be prevented.

It is believed that the arsenic compounds can reduce the eotaxin amount and decrease the eosinophils accumulation on the affected parts of the human or animal. A preferred method of administering the arsenic compounds is parenterally, as by injection, infusion, topical application or inhalation. Thus, the present method provides a therapy for bronchial asthma in the lung, eosinophil-associated intranasal inflammation, including inflammation of the paranasal sinuses, and eosinophil-associated inflammation of the eye, such as vernal conjunctivitis. Preferred arsenic compounds are inorganic arsenic compounds, inclusive of arsenic trioxide (As₂O₃) and arsenic hexoxide (As₄O₆).

The present method is believed to involve counteracting or preventing the symptomologies caused by eosinophil via the parenteral administration of arsenic compounds to the afflicted or susceptible human or animal. For example, the present invention provides a therapy for bronchial asthma and the other hypersensitivity diseases of the respiratory tract, by intravenous injection or infusion of an arsenic compound, which is preferably arsenic trioxide. The arsenic compounds in turn are able to decrease eosinophil amount in situ. Topical administration of arsenic compounds, e.g., in eyedrops, can relieve the symptoms of conditions due to eosinophil-associated inflammation of the eye, such as vernal conjunctivitis.

The present invention also provides a unit dosage form comprising an amount of arsenic compounds optimal to treat bronchial asthma of human and animal by some conversion methods.

The present invention demonstrates that arsenic compounds are effective in treating asthmatic disease because they do reduce the hyperactivity to brochoconstriction. When asthma attacks, increasing in airway resistance is a major pathophysiologic phenomenon. Tests of the effect of the arsenic trioxide on the increase in respiratory resistance (bronchospasm) due to methacholine administration to BALB/c mice showed that arsenic trioxide does decrease the airway responsiveness.

The term "arsenic-susceptible blast-like cell" used herein refers to a cell that is capable to division and susceptible to an arsenic compound. The cell may be a leukocyte, a lymphocyte, a mast cell, a T cell, a B cell, a hemopoietic stem cell, a fibroblastic cell or a parasite. The parasites in human body proliferate by asexual reproduction, therefore, the parasites like foreign cells in human body and are the targets for the arsenic compound.

The term "arsenic-susceptible blast-like cell related disease" used herein refers to a hypersusceptible disease, an immune or autoimmune disease, a fibroblast-related disease, an inflammation disease or a parasitic disease.

The term "arsenic compound" as used herein refers to an arsenic compound in variety of known forms, for example, arsenic can be administered as a salt, an organic or inorganic complex, an organic chelate, an organic compound or an organic or inorganic solution. It is preferred that the form be chosen to reduce toxicity and improve efficacy. The inorganic salt forms of arsenic are preferred. For example, inorganic salts such as arsenic arsenic oxide, triiodide, arsenic(III)bromide, arsenic(III)chloride, arsenic pentoxide, arsenic trioxide, Fowler's solution (potassium arsenite), sodium arsenite, and calcium arsenite may be used. Arsenic oxide may be arsenic hexoide or arsenic trioxide. Arsenic trioxide is most preferred. Further, arsenic sulfides may be used such as arsenous sulfide, arsenic sulfide, arsenic pentasulfide, tetraarsenic trisulfide and tetraarsenic pentasulfide. Without being limited by any theory, certain of these arsenic compounds may be prodrugs to an active species. Generally, the skilled artisan will recognize that the form of arsenic to be used should be therapeutically effective without unreasonable toxicity. The toxicity is dependent upon the dose, the dosage form, the mode of administration and frequency of dosing. Generally, the skilled artisan can chose from the following known forms of arsenic: arsenic halides, arsenic oxides, arsenic acids, arsenic sulfides, and the like.

The term "medicament" as used herein refers to a medicine that is formed as powder, injections, tablets or capsules.

This invention is illustrated further rather than limited by the following examples. All of the references listed in the application are hereby incorporated by reference.

### Examples

EXAMPLE 1:Arsenic trioxide induces apoptosis of T cells and peripheral blood mononuclear cells (PBMC) *in vitro*
1. The isolation procedure using Ficoll Plaque for exploiting the different densities of RBCs and PBMCs to separate them;
2. The detection of apoptosis by PI staining through flowcytometry; and
3. The detection of apoptosis within different subsets of PBMCs by the combination of mAb-fluorescein tandem staining and the treatment of Phiphilux through flowcytometry. Phiphilux is a substrate of caspase-3, composed of a specific oligo-peptide with its sequence subjected to functional caspase-3 cutting and, at each end of the peptide, is covalently coupled to two identical fluorophores.

In the PI-based study, under different concentrations of arsenic trioxide (0, 0.5, 2.5, 5, or 10 µM), the percentages of cell death (within all PBMCs) in 24hr and 48hr were calculated and the results are shown respectively in Table 1.1.

**Table 1.1: the percentages of cell death in 24hr and 48hr**

| Concentration | 24hr %cell death | 48hr %cell death |
|---|---|---|
| Control | 6.2 ±0.6% | 7.3±0.8% |
| 0.5µM | 7.3 ± 1.2% | 7.6± 0.6% |
| 2.5µM | 13.7±1,6 | 15.2±.2.3 % |
| 5.0µM | 14.6±2.1% | 17.2±1.9% |
| 10µM | 17.8±1.8% | 20.3±2.6% |

There was no dose-dependent relationship from 2.5 to 10 µM in 24 or 48hr (however, there was indeed a slight increase in the death level for the 48 hr group), so some specific types of PBMCs might be more susceptible to arsenic trioxide than others.

Furthermore, according to that suspect, the apoptotic cell death within subsets of PBMCs was investigated by detecting caspase-3 activity within gated PBMC groups according to their surface markers (i.e. for T lymphocytes were recognize as "CD3⁺", B lymphocytes as "CD19⁺"), the results showed that most T cells (96%) were dead when treated with 5µM arsenic trioxide for 48 hour (Table 1.2). Thus, T lymphocytes are more susceptible to arsenic trioxide according the flow data.

**Table 1.2: Levels of T cell apoptosis (%) after 48 hr treatment**

| Control | 34.5 |
|---|---|
| 1µM | 36.1 |
| 5µM | 96.2 |

EXAMPLE 2: Biological effect of arsenic trioxide on cultured human fibroblasts showed highlighted on cell cycle and arsenic compound treated fibroblasts underwent both apoptosis and necrosis.

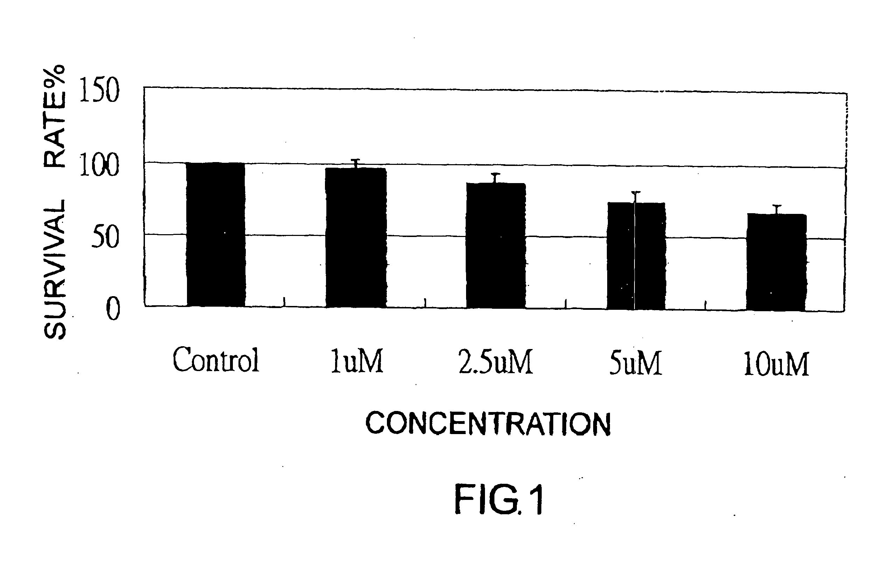

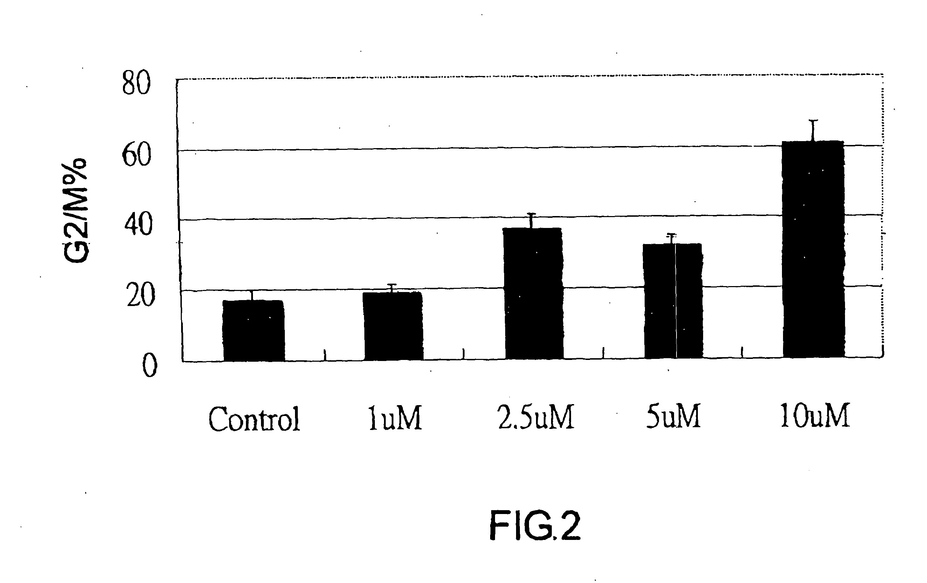

In cultured human fibroblasts exposed for 24 hr to 1 to 10 □M arsenic trioxide and the fibroblasts were assayed by MTT assay, flowcytometry and western blot and promoter leuciferase assay to find out the biological effect of arsenic trioxide on cell cycle of cultured human fibroblasts, and though it was found that arsenic trioxide can cause fibroblasts cell cycle arrest especially in G2/M, on the molecular level, p53 seems to be bound with cell cycle arrest caused by arsenic trioxide (Fig. 1 and 2).

Fibroblasts were treated with different concentrations of arsenic trioxide for 24 hours, cells were fixed and the apoptotic bodies were detected by DAPI staining. The data showed that there were no obvious apoptosis in control, 1□M and 2.5□M groups. In contrast, there were obvious apoptotic bodies present in 5□M and in the condition of 10□M, fibroblasts underwent both apoptosis and necrosis.

### EXAMPLE 3: Method for the treatment of asthma and like hypersensitivity diseases by administration of arsenic compounds

### 3.1 Materials and Methods

BALB/c mice (female, 6-8 weeks) were immunized with ovalbumin (OVA) at day 0, 14, 21 and 28. 38 days later, animals were injected with arsenic trioxide (ATO) i.p. for 7 days and antigen challenges with OVA were performed at day 42, 43, 44. At day 45, the airway enhanced pause (PenH) values were measured. 48 hours after the last antigen challenges, the mice were sacrificed, the trachea cannulated, and bronchoalveolar lavage (BAL) was performed by four repeatedlavages with balanced salt solution (HBSS) injected into the lungs via the trachea. Total cell counts were performed with a hemocytometer after staining with trypan blue, and then cytospins were prepared, and a differential count of 300 cells per slide was performed. To obtain the absolute number of each leukocyte subtype in the lavage, the percentage of each cell type was multiplied by the total number of cells that were recovered from the bronchoalveolar lavage fluid (BA LF). Eotaxin in the BALF was measured via ELISA. Four groups of mice were treated as Table 3.1.

Group A mice were challenged by PBS only and then treated by PBS, too. Group B mice were immunized by ovalbumin and then treated by PBS only. Group C mice were immunized by ovalbumin and then treated by arsenic trioxide in a dosage of 2.5mg per kg for mice. Group D mice were challenged by ovalbumin as group B and C and then treated by arsenic trioxide in a dosage of 5 mg/kg.

**Table 3.1**

| | Antigen challenge | Treatment |
|---|---|---|
| Group A | PBS | PBS |
| Group B | OVA | PBS |
| Group C | OVA | ATO 2.5 mg/kg |
| Group D | OVA | ATO 5 mg/kg |

### 3.2. Results

### 3.2.1 OVA stimulation index

After antigen challenges, mice serum were prepared and OVA-specific IgE values in serum were detected for evidence of immunization. The data in Table 3.2 show the mice of group B, C, and D were successfully immunized by ovalbumin because the serum levels of OVA-specific IgE were significantly elevated in comparison with mice in group A, the control group.

**Table 3.2 OVA-specific IgE (SI.) in serum**

| | Mean | SD |
|---|---|---|
| Group A | 0.30 | 0.09 |
| Group B | 2.06 | 0.81 |
| Group C | 2.20 | 0.70 |
| Group D | 1.96 | 0.31 |

### 3.2.3 Eotaxin in BALF

Table 3.3 shows the mean value and standard deviation of eotaxin in BALF of mice within each group. It was demonstrated that the arsenic treatment does diminish the eotaxin level after antigen challenge especially in a dosage of 2.5 mg/kg, because there is a marked and meaningful difference between group B and group C (p < 0.05).

**Table 3.3 mean value and standard deviation of eotaxin (pg/ml) in BALF of mice within each group**

| | Mean | SD |
|---|---|---|
| Group A | 14.42 | 8.39 |
| Group B | 20.90 | 4.97 |
| Group C | 12.38* | 3.92 |
| Group D | 16.00 | 4.06 |

| | | |
|---|---|---|
| *p<0.05, compared with group B (OVA-PBS) | | |

### 3.2.3. Eosinophils in BALF

The white blood cells in BALF were counted and differentiated. The mean concentration and standard deviation of each cell type in each group are shown in the Table 3.4. Every kind of white cell was increased in immunized groups when compared with the non-immunized group. However, arsenic trioxide treatments (group C and D) had diminished significantly the eosinophil number but enhanced the macrophage and neutrophil numbers in contrast with non-treatment group (group B). It can be concluded that eosinophil can reduce by arsenic trioxide treatment and the result is consistent with eotaxin level in BALF.

**Table 3.4 mean concentration and standard deviation of each cell type in each group (× 1000 cells/ml)**

| | Macrophage | Eosinophil | Neutrophil | Lymphocyte* |
|---|---|---|---|---|
| Group A | 135.6 ± 54.5^{#} | 3.2 ± 2.1 | 8.4 ± 4.9 | 12.9 ± 8.2 |
| Group B | 243.8 ± 69.3 | 105.3 ± 60. | 777.1 ± 32.8 | 37.8 ± 16.3 |
| Group C | 621.0 ± 199.6 | 28.6 ± 13.0 | 200.7 ± 75.2 | 37.0 ± 9.4 |
| Group D | 499.1 ± 131.6 | 33.2 ± 12.5 | 244.0 ± 72.6 | 28.7 ± 13.6 |

| | | | | |
|---|---|---|---|---|
| *Basophil count is so negligible that the numbers are not shown. ^{#}Values given as mean ± SD | | | | |

### 3.2.4. Airway responsiveness in arsenic treated animal

The mice were challenged with methacholine (Mch.) in an increasing concentration and the airway constriction of the mice was measured by using pulmonary function test, whereafter the data of Table 3.5 was obtained. This data shows that airway hyper-reactivity of mice was successfully blocked by arsenic trioxide treatment especially in a dosage of 2.5 mg/kg, when comparing the data between group B and group C. The result is also consistent with the results of eotaxin and eosinophil in BALF.

**TABLE 5 - the mice in an increasing concentration and the result of the airway constriction of the mice by pulmonary function test**

| Mch(mg/ml) | 6.25 | 12.5 | 25 | 50 |
|---|---|---|---|---|
| Group A | 2.20 ± 0.28^{#} | 8.22 ± 1.22 | 11.99 ± 1.50 | 14.21 ± 1.06 |
| Group B | 2.50 ± 0.40 | 8.00 ± 0.93 | 14.52 ± 1.42 | 19.15 ± 3.35 |
| Group C | 3.62 ± 0.47 | 7.83 ± 1.13 | 10.15 ± 0.84* | 9.83 ± 1.17* |
| Group D | 2.55 ± 0.35 | 8.82 ± 0.88 | 11.57 ± 1.52 | 12.51 ± 1.90 |

| | | | | |
|---|---|---|---|---|
| ^{#}Values given as mean ± SD * p< 0.05, compare with Group B (OVA-PBS) | | | | |

### EXAMPLE 4: Method for the treatment of systemic lupus

erythematosus and like autoimmune diseases by administration of arsenic compounds

### 4.1 Arsenic trioxide preparation

Commercial arsenic trioxide injection solution (Asadin^{®}, 10mg/10ml/vial) was chosen and diluted by PBS solution.

### 4.2 Anti- human systemic lupus erythematosus activity in vivo

*In vivo* studies on the activity of arsenic compound were carried out by using an experimental animal model of human systemic lupus erythematosus. NZB/NZW denotes the mouse strain that spontaneously develops an autoimmune syndrome having notable similarities to human systemic lupus erythematosus. Autoimmune disease in NZB/NZW mice is characterized by antinuclear antibodies (IgG) production about 4 months of age, and glomerulonephritis with proteinuria is developed when at 9 months of age, and that is the major cause of death (uremia).

At 24 weeks of age, mice (3 groups) were treated 3 times per week with PBS (Group 1 as positive control), 2.5 mg/kg of As₂O₃ (Group II), or 5 mg/kg of As₂O₃ (Group III) administered intraperitoneally. The treatment was stopped at 28 weeks of age. Mice were observed daily for clinical signs of disease and for mortality and were bled every 4 weeks for determination of anti-DNA antibody production.

Standard ELISA assay to measure the serum levels of anti-DNA antibodies was performed as follows. The plates were coated 10□g/mL mBSA 4°C overnight before coating dsDNA and ssDNA diluted with PBS to concentration of 7.5□g /mL. ssDNA was prepared by boiling for 20min then placed on ice immediately for 20 min. After overnight incubation at 4°C, the plates were washed and blocked with gelatin-PBS solution. The serum was diluted and then added to the appropriate wells, which were then incubated at 37°C for 45 min. Horseradish peroxidase-conjugated anti-mouse □-chain-specific antibodies were added. After incubation at 37°C for 50min, then room temperature for 10 min, 2,2'-azino-bisC3-ethylbenzthiazoline-6-sulphonic acid (ABTS) solution was used as substrate and the absorbance was measured at 405 nm. The level of anti-DNA IgG is presented as ELISA units (EU/ml) compared with mAb 10F10. The OD value generated by 74 ng/ml of 10F10 Ab was defined as 1 EU/ml for anti-DNA IgG. The level of anti-nucleosome IgG was calculated as EU by comparison with 10F10 Ab. The absorbance value generated by 231 ng/ml of 10F10 Ab was defined as 1 EU/ml of anti-nucleosome IgG. The results of anti-ssDNA are shown in Table 4.1 and the results of anti-dsDNA are shown in Table 4.2.

**Table 4.1 IgG Anti-ssDNA titer (ELISA Unit)**

| Treatment | Time after treatment (weeks) | | | |
|---|---|---|---|---|
| | Before treatment | 4 | 8 | 12 |
| PBS | 0.171 | 0.333 | 0.465 | 0.105 |
| 2.5mg/kg ATO | 0.155 | 0.347 | 0.400 | 0.105 |
| 5mg/kg ATO | 0.176 | 0.303 | 0.296 | 0.201 |

**Table 4.2 IgG Anti-dsDNA titer (ELISA Unit)**

| Treatment | Time after treatment (weeks) | | | |
|---|---|---|---|---|
| | Before treatment | 4 | 8 | 12 |
| PBS | 0.280 | 0.623 | 0.679 | 0.381 |
| 2.5mg/kg ATO | 0.262 | 0.572 | 0.464 | 0.442 |
| 5mg/kg ATO | 0.362 | 0.285 | 0.357 | 0.702 |

As shown in Table 4.1 and Table 4.2, sera obtained from mice treated with arsenic trioxide showed significantly lower anti-DNA antibody levels compared with mice treated with PBS alone. A higher concentration of arsenic trioxide solution had more effect than lower concentration. Because anti-DNA antibody levels are strongly correlated with SLE disease activity, these results demonstrate that administration of arsenic trioxide provides a new approach for the treatment of human SLE.

Urine protein levels were determined by colorimetric analysis using dipsticks (Multistix^{®}, Bayer). Severe glomerulonephritis was defined as proteinuria more than 1g/L (2+) by urine dipstick. The results are shown in Table 4.3.

**Table 4.3 Mice with severe glomerulonephritis (%)**

| Treatment | Time (weeks) | |
|---|---|---|
| | 37 | 40 |
| PBS | 25 | 100 |
| 2.5mg/kg ATO | 16.67 | 40 |
| 5mg/kg ATO | 25 | 25 |

Data reported in Table 4.3 show that a marked delay in the onset of severe proteinuria was achieved in animals treated with 2.5mg/kg ATO and 5mg/kg ATO in comparison with untreated animals. Whereas although 100% of control mice had developed proteinuria, some As₂O₃-treated mice did not develop proteinuria, and a significant percentage of these mice maintained normal renal function withoutevidence of proteinuria. Treatment with arsenic trioxide induced an evident reduction in the glomerulonephropathic progression, in comparison to the control group.

Coincident with ameliorating the clinical signs of severe immune complex nephritis, a dramatic prolongation of survival was observed. The results are shown in Table 4.4.

**Table 4.4 survival rate (%) of the mice treated with As₂O₃**

| Treatment | Time (weeks) | | | | | | | |
|---|---|---|---|---|---|---|---|---|
| | 0 | 22 | 31 | 38 | 39 | 40 | 41 | 42 |
| PBS | 100 | 100 | 66.7 | 50 | 50 | 16.7 | 0 | 0 |
| 2.5mg/kg | 100 | 100 | 100 | 83.3 | 66.7 | 66.7 | 66.7 | 50 |
| ATO | | | | | | | | |
| 5mg/kg | 100 | 100 | 66.7 | 66.7 | 66.7 | 66.7 | 66.7 | 66.7 |
| ATO | | | | | | | | |

Data reported in Table 4.4 show that 50% of arsenic trioxide treated mice were still alive after 43 weeks, while no animals treated with placebo (PBS) were still alive.

### EXAMPLE 5: Arsenic compounds for the treatment of parasitic diseases

### 5.1 Arsenic trioxide preparation

Commercial arsenic trioxide injection solution (Asadin^{®}, 10mg/10ml/vial) was chosen. The molecular weight of As₂O₃ is 197.84.

### 5.2 Malaria inhibition test

FCC1/NH strain (from Hainan, P.R.C.) of P, falciparum was used. The strain of the parasite was cultured by culture media mainly composed of RPMI-1640(GBCO), HEPES (GBCO), NaHCO₃, and AB type serum. In a given experiment, Petri dish cultures were diluted with culture medium containing an amount of noninfected type B human erythrocytes to obtain a culture with a final parasitemia of about 1%. The resulting culture was ready for addition to microtitration plates with 96 flat-bottom wells.

The final volume added to each of the 96-well microtitration plates was 200 µl, consisted of 20 µl of complete medium with or without the drug (arsenic trioxide) and 180 µl of the parasitized culture. The microtitration plates were incubated in 5% CO₂ for an additional 48 hr, at 37□C. The suspensions were discarded and infection rate of RBC in each well was calculated by microscope examination after Giemsa staining. When drugs interfere, an inhibitory dose of 50% (IC₅₀) and 90% (IC₉₀) can be calculated. A series of experiments is shown below.

### 5.2.1

The malaria inhibition experiment was performed in 4 lanes for each arsenic trioxide dose, and the initial infectious rate is 1.8%. After 48 hours of incubation, 5000 RBCs in each well were checked by microscope. The inhibition rate is shown in Table 5.1.

**Table 5.1. Inhibition rate to malaria**

| Dose (uM) | Log₁₀ of Dose (X) | Infection rate (%) | Inhibition rate (%) | Probability unit (Y) |
|---|---|---|---|---|
| 0.25 | -0.6021 | 42.3 | 57.7 | 5.1942 |
| 0.5 | -0.3010 | 24.5 | 75.5 | 5.6903 |
| 1.0 | 0 | 19.5 | 80.5 | 5.8596 |
| 2.0 | 0.3010 | 13.7 | 86.3 | 6.0939 |
| 4.0 | 0.6021 | 9.3 | 90.7 | 6.3225 |
| 8.0 | 0.9031 | 6.8 | 93.2 | 6.4909 |
| | Y=0.8177x+5.8188 | | r²=0.9651 | |
| IC₅₀=0.10uM=19.78ng/ml | | 95% confidence limit (11.43∼34.2ng/ml) | | |
| | IC₉₀=3.68uM=728.05ng/ml | | 95% confidence limit | |
| (420.84~1259.53ng/ml) | | | | |

### 5.2.2

Another malaria inhibition experiment was performed in 4 lanes for each arsenic trioxide dose, and the initial infectious rate is 1.5%. After 48 hours of incubation, 5000 RBCs in each well were checked by microscope. The inhibition rate is shown in Table 5.2.

**Table 5.2. Inhibition rate to malaria**

| Dose (uM) | Log₁₀ of Dose (X) | Infection rate ( % ) | Inhibition rate (%) | Probability unit (Y) |
|---|---|---|---|---|
| 0,031 | -1.509 | 79.9 | 20.1 | 4.1619 |
| 0.063 | -1.201 | 73.7 | 26.3 | 4.3659 |
| 0.125 | -0.903 | 68.3 | 31.7 | 4.5239 |
| 0.25 | -0.6021 | 54.9 | 45.1 | 4.8743 |
| 0.5 | -0.3010 | 44.4 | 55.6 | 5.1408 |
| 1.0 | 0 | 17.9 | 82.1 | 5.9192 |
| 2.0 | 0.3010 | 12.9 | 87.1 | 6.1311 |
| 4.0 | 0.602 1 | 11.2 | 88.8 | 6.2160 |
| 8.0 | 0.9031 | 5.1 | 94.9 | 6.6352 |
| | Y=1.0904x+5.6582 | | r²=0.9706 | |
| IC₅₀=0.25uM=49.28ng/ml | | 95% confidence limit ((28.00~86.73ng/ml) | | |
| | IC₉₀=3.73uM=737.94ng/ml | | 95% confidence limit | |
| (419.28~1298.77ng/ml) | | | | |

### 5.2.3

The other malaria inhibition experiment was performed in 12 lanes for each arsenic trioxide dose, and the initial infectious rate is 1.1%, After 48 hours of incubation, 5000 RBCs in each well were checked by microscope. The inhibition rate is shown in Table 5.3.

**Table 5.3. Inhibition rate to malaria**

| Dose (uM) | Log₁₀ of Dose (X) | Infection rate (%) | Inhibition rate (%) | Probability unit (Y) |
|---|---|---|---|---|
| 0.5 | -0.3010 | 27.4 | 72.6 | 5.6008 |
| 1.0 | 0 | 12.9 | 87.1 | 5.1311 |
| 2.0 | 0.3010 | 8.5 | 91.5 | 6.3722 |
| 4.0 | 0.6021 | 3.6 | 96.3 | 6.7866 |
| 8.0 | 0.9031 | 2.0 | 98.0 | 7.0537 |
| | Y=1.1832x+6.0328 | | r²=0.9849 | |
| IC₅₀=0.13uM=25.72ng/ml | | 95% confidence limit (18.91~34.9ng/ml) | | |
| IC₉₀=0.96uM=189.93ng/ml | | 95% confidence limit ( 139.65~258.3ng/ml) | | |

### 5.2.4

The other malaria inhibition experiment was performed in 6 lanes for each arsenic trioxide dose, and the initial infectious rate is 1.1%, After 48 hours of incubation, 5000 RBCs in each well were checked by microscope. The inhibition rate is shown in Table 5.4.

**Table 5.4. Inhibition rate to malaria**

| Dose (uM) | Log₁₀ of Dose (X) | Infection rate (%) | Inhibition rate (%) | Probability unit (Y) |
|---|---|---|---|---|
| 0.5 | -0.3010 | 24.5 | 75.5 | 5.6903 |
| 1.0 | 0 | 15,7 | 84.3 | 6.0027 |
| 2.0 | 0.3010 | 9.0 | 91.0 | 6.3408 |
| 4.0 | 0.6021 | 4.0 | 96.0 | 6.7507 |
| 8.0 | 0.9031 | 1.9 | 98.1 | 7.0749 |
| | Y=1.5032x+5.8726 | | r²=0.9495 | |
| IC₅₀=0.32uM=63.31ng/ml | | 95% confidence limit (40.32∼99.4ng/ml) | | |
| IC₉₀=2.09uM=413.48ng/ml | | 95% confidence limit | | |
| (263.06~258.30ng/ml) | | | | |

### 5.3 Anti- Trypanosoma test

Arsenic trioxide (Asadin^{®}, 10mg/10ml/vial) was used to check the toxicity on the Trypanosoma brucei. Viable cell count was microscopically monitored. The complete killing effect over 7 days was observed at dose of 0.1mg/ml/day for 3 days. One dose regimen of 0.3mg/ml showed less killing potency at least one log difference than 3 doses regimen. This is one third of recommended dosage of Melarsoprol (an Anti-Trypanosoma drug). Moreover, cytology observation noticed specific death morphology on cell toxicity. Cells became thread-like instead of the usual small, round death cell. Further cytology examination on the death cells will be examined. In conclusion, arsenic trioxide can kill cultured Trypanosoma brucei at dosage comparable, if not better, to that of Melarsoprol.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. Use of an arsenic compound for the manufacture of a medicament for the treatment of arsenic-susceptible blast-like cell related diseases.

2. The use as claimed in claim 1, wherein said arsenic compound is administered parenterally.

3. The use as claimed in claim 1, wherein said arsenic-susceptible blast-like cell related disease is a hypersusceptible disease, an immune or autoimmune disease, a fibroblast-related disease, an inflammation disease or a parasitic disease.

4. The use as claimed in claim 3, wherein said immune or autoimmune disease is a connective tissue disease, an autoimmune thyroid disease, a neuromuscular junction autoimmune disease, a autoimmune gastrointestinal disease, autoimmune hepatitis, primary biliary cirrhosis, primary sclerosing cholangitis, autoimmune carditis or arteritis.

5. The use as claimed in claim 4, wherein said immune or autoimmune disease is systemic lupus erythematosus, rheumatoid arthritis, sclerosis, Graves' disease, Myasthenia Gravis, multiple sclerosis, ulcerative colitis or Crohn's disease.

6. The use as claimed in claim 3, wherein said hypersusceptible disease is bronchial asthma, hypersensitivity pneumonitis, diffuse pulmonary interstitial fibrosis, allergic rhinitis, eosinophil-associated nasal inflammation, vernal conjunctivitis or urticaria.

7. The use as claimed in claim 3, wherein said inflammation disease is pneumoconiosis, osteomyelitis, leprous nodule, syphilis, tuberculosis, hepatitis, tumor formation or a chronic obstructive pulmonary disease.

8. The use as claimed in claim 3, wherein said fibroblast-related disease is hepatic fibrosis, pulmonary fibrosis, cutaneous and subcutaneous fibrosis or systemic fibrosis.

9. The use as claimed in claim 8, wherein said fibroblast-related disease is liver cirrhosis, pneumoconiosis, tuberculosis, severe acute respiratory syndrome (SARS), adult (acute) respiratory distress syndrome(ARDS), chronic obstructive pulmonary disease (COPD), scarring, keloid, psoriasis, cystic fibrosis or neurofibromatosis.

10. The use as claimed in claim 3, wherein said parasitic disease is malaria or trypanosomiasis.

11. The use as claimed in claim 1, wherein said arsenic-susceptible blast-like cell is a leukocyte, a peripheral blood mononuclear cell, a fibroblast and a parasite.

12. The use as claimed in claim 1, wherein the arsenic compound is administered is in a dosage of between 0,001 micromolar to 20 micromolar.

13. The use as claimed in claim 12, wherein the arsenic compound is administered is in a dosage of between 0.1 micromolar to 15 micromolar.

14. The use as claimed in claim 13, wherein the arsenic compound is administered is in a dosage of between 0.1 micromolar to 10 micromolar.
